# EUROPEAN PATENT APPLICATION

(11) **EP 4 783 179 A1**
(43) Date of publication of application: **29.07.2026**
(21) Application number: 26151659.5
(22) Date of filing: 13.01.2026
(51) Int. Cl.: G16C 60/00, G01N 3/00, G06F 30/27

(54) **METHOD FOR PREDICTING FRACTURE TOUGHNESS FAILURE ON METALLIC PARTS**

(30) Priority: 23.01.2025 US 202519034821
(71) Applicant: The Boeing Company, Arlington, VA 22202 (US)
(72) Inventor: WRENN, Katy Marie, Arlington, VA 22202 (US); JONES, Lindsay Leigh Waite, Arlington, VA 22202 (US); KURPIUS, Katey Marie, Arlington, VA 22202 (US); DUVALL, Tracy Lynn, Arlington, VA 22202 (US)
(74) Representative: Sandri, Sandro

(57) **Abstract**

A method of predicting the probability of fracture toughness failure of a metallic part. The method includes receiving static mechanical property data for a metallic part. The method further includes determining a predicted probability of fracture toughness failure for the metallic part using a selected statistical model. The method further includes flagging, to perform fracture toughness testing, the metallic part based on the predicted probability of fracture toughness failure being over a threshold.

## Description

### TECHNOLOGICAL FIELD

The present disclosure relates generally to the field of fracture toughness analysis. More specifically, the present disclosure relates to predicting the fracture toughness failure of metallic parts with respect to engineering requirements.

### BACKGROUND

Fracture toughness is a material property that describes a material's ability to resist fracture when a crack is present. Fracture toughness testing is used to evaluate materials' durability and reliability, especially in applications where structural integrity is paramount, for example, in metallic materials used to build an aircraft. A fracture toughness test may determine whether a metallic part has passed or failed engineering requirements. Aircraft parts and subparts undergo tests to ensure that the parts pass certain engineering requirements that dictate minimum mechanical properties for each part of the aircraft.

The existing techniques for determining fracture toughness include performing the mechanical test on all parts in question. The drawback to testing every part is that fracture toughness testing is expensive, time-consuming, and requires special equipment conducted by approved testing labs. This could potentially delay resolving nonconformance issues with the parts.

### SUMMARY

One aspect of the present disclosure provides a method of predicting the probability of fracture toughness failure of a metallic part with respect to engineering requirements. The method includes receiving static mechanical property data for a metallic part. The method further includes determining a predicted probability of fracture toughness failure for the metallic part using a selected statistical model. The method further includes flagging, to perform fracture toughness testing, the metallic part based on the predicted probability of fracture toughness failure being over a threshold.

In some aspects, the selected statistical model is a generalized linear model or logistic regression model.

In some aspects, developing the selected statistical model using a training dataset and a test dataset with the training dataset and the test dataset comprising of paired static mechanical property data and fracture toughness data.

In some aspects, a full dataset is split into 75% training dataset and 25% testing dataset.

In some aspects, the static mechanical property data comprises pass and fail fracture toughness data of metallic parts that have previously undergone fracture toughness testing based on engineering requirements.

In some aspects, the method further includes selecting the metallic part from a heat treat lot comprising multiple metallic parts.

In some aspects, the method further includes evaluating the selected statistical model using user input.

In some aspects, the methods further include setting the threshold based on user input obtained prior to predicting a probability of fracture toughness failure.

In some aspects, flagging the metallic part is responsive to determining that the metallic part is likely to fail an engineering requirement for fracture toughness.

In another aspect the disclosure provides a method of predicting fracture toughness of a metallic part. The method includes selecting a metallic part. The method further includes receiving static mechanical property data for the metallic part. The method further includes determining a predicted probability of fracture toughness failure using a model with the static mechanical property data as input. The method further includes outputting a failure indicator for the metallic part based on the probability of fracture toughness being over the threshold.

In some aspects, the static mechanical property data indicates how the metallic part reacts to external forces, such as pulling, compressing or shearing.

In some aspects, the model is developed using stored paired static mechanical property data and fracture toughness data from previously tested metallic parts.

In some aspects, the metallic part is selected from a heat treat lot comprising multiple metallic parts.

In some aspects, the model is a statistical model.

In some aspects, the model produces a predicted probability of fracture toughness failure.

In some aspects, logistic regression is used to model the binary outcome of a fracture toughness test with respect to an engineering requirement.

In another aspect the present disclosure provides a non-transitory computer-readable medium storing a computer program product for controlling a computing device, the computer program product comprising software instructions that, when run on the computing device cause the computing device to receive static mechanical property data for a part that has not undergone fracture toughness testing. The software instruction further causes the computing device to determine a predicted probability of fracture toughness failure using a selected statistical model with the static mechanical property data as input. The software instruction further causes the computing device to flag, for further fracture toughness testing, the metallic part based on the probability of fracture toughness failure being over a threshold.

In some aspects, the metallic part is selected from a heat treat lot including multiple parts.

In some aspects, the static mechanical property data for a metallic part comprises pass and fail fracture toughness data of previously undergone-fracture toughness testing.

In some aspects, the static mechanical property data comprises data indicating compression, ultimate tensile strength, yield strength, percent elongation, percent reduction of area, and hardness after aging of a tested or untested metallic part.

The features, functions and advantages that have been discussed can be achieved independently in various aspects or may be combined in yet other aspects, further details of which can be seen with reference to the following description and the drawings.

### BRIEF DESCRIPTION OF THE DRAWINGS

Figure 1 is a flowchart illustrating a method for selecting and fitting a best statistical model.
Figure 2 is a flowchart illustrating a method for selecting and fitting a best statistical model.
Figure 3 is a flowchart illustrating a method for predicting fracture toughness failure.
Figure 4 is a flowchart illustrating a method for predicting fracture toughness failure.
Figure 5 is a flowchart illustrating a method for predicting fracture toughness failure.
Figure 6 is a flowchart illustrating a method for predicting fracture toughness failure.
Figure 7 is a schematic block diagram illustrating an example of a computing device.

### DETAILED DESCRIPTION

The present application is directed to predicting the probability of fracture toughness failure on untested metallic parts using a statistical model. The statistical model is developed using static mechanical property data from previously tested metallic parts. The static mechanical properties of the metallic part are used as inputs into the statistical model to predict whether the metallic part is likely to pass or fail an actual fracture toughness test.

The process includes selecting statistical models to predict the probability of fracture toughness failure. Statistical models are developed using machine learning with a full dataset, including static mechanical property data, that is divided into training and test datasets. The training dataset includes data to fit a model by identifying patterns within the data. In contrast, the test dataset is a separate independent dataset to evaluate the performance of the trained model. Model fitting measures how well a model describes a set of observations. The fitting process involves adjusting the statistical model's parameters to optimize the match between the model's predictions and the actual data.

The full dataset is divided into different proportions to produce a training and test dataset. In some examples, the full dataset is partitioned such that 75% is used for the training dataset and 25% for the test dataset. In another example, 80% of the full dataset is used for the training dataset and the remaining 20% is used for the test dataset.

The training and test dataset includes static mechanical property data related to the fracture toughness of a part. Examples of static mechanical property data include but are not limited to tensile, compression and hardness, and other critical properties of tested metallic parts. In some examples, the dataset includes pass or fail indicators with respect to fracture toughness engineering requirements. In some examples, a stratified sampling method is used to ensure the ratio of passes to failures with respect to fracture toughness in the training and test datasets is similar to that of the full dataset.

The statistical model is a logistic regression model fit to the training dataset which includes terms for static mechanical properties (compression, ultimate tensile strength, yield strength, percent elongation, percent reduction of area, and hardness after aging), part number effects, and two-way interaction effects between the static mechanical properties themselves and the part number effects. In some examples, the training dataset includes static mechanical property data and fracture toughness data. Static mechanical property data is data that describes how a material reacts to external forces, such as pulling, compression or shearing. Fracture toughness data is data that indicates the amount of stress required to propagate a preexisting flaw in the material.

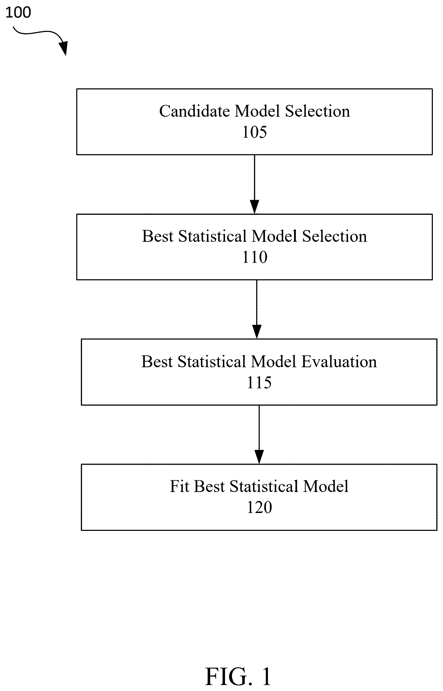

Figure 1 illustrates a method (100) for selecting and fitting the best statistical model used to predict the probability of fracture toughness failure. In some examples, the statistical model is a generalized linear model or logistic regression used to model binary outcomes, such as pass/fail outcomes. The statistical model may also use multivariate regression for predicting fracture toughness failure magnitude.

Candidate models are selected from statistical models (block 105). The selection process entails selecting statistical models that best represent the training dataset.

In some examples, the selection process uses the training dataset with a 10-fold cross-validation approach in conjunction with a stepwise model selection procedure. Specifically, the training dataset is partitioned into ten groups. For the first fold, nine of the ten groups (referred to as the cross-validation training set) are used to train the model, and the remaining group is used as an assessment set (referred to as a cross-validation assessment set). The statistical model (which includes the main effects for the mechanical properties, part number, and two-way interactions) is fit to the cross-validation training set. In some examples, stepwise model selection using Bayesian Information Criterion (BIC) and Akaike Information Criterion (AIC) are used to select the most relevant independent variables resulting in the best candidate model for the cross-validation fold. The AIC and BIC-selected models are evaluated on the cross-validation training and cross-validation assessment sets by computing the area under the Receiver Operator Characteristic (ROC) curve, a model performance metric. This process is repeated so that a different set of nine groups is used each time to train and select the best statistical model, and the group that is left out is used for assessing the models.

The best statistical model is selected among the candidate models from the cross-validation and stepwise model selection procedure (block 110). In some examples, the best of the candidate models are chosen based on performance across the cross-validation training and assessment sets and their frequency of selection during the cross-validation process.

Once best of the candidate models are chosen, the entire training dataset is used to fit the logistic regression models. The best statistical models are evaluated on the entire training set and the independent test data (block 115). In some examples, the ROC curves of the test dataset and training dataset are assessed, and the area under the ROC curve (AUC) is computed to assess model performance. A ROC curve is used to assess the performance of a binary classifier across different classification thresholds and can be visually and quantitatively assessed. In particular, the closer the ROC curve is to the upper left side of the graph, the better the model is at discriminating between pass and fail classes compared to a ROC curve that is closer to the 45-degree line. The area under the ROC curve is used to measure overall model performance. AUC can be between zero and one with an AUC close to one indicating a model with good diagnostic capability. In this example the ROC and AUC performance metrics are used for assessing model performance, however this disclosure is not limited to the use of ROC and AUC, other performance metrics may be used to assess model performance. If the best statistical model is deemed adequate based on model performance and user input, all data is used to fit the best statistical model (block 120).

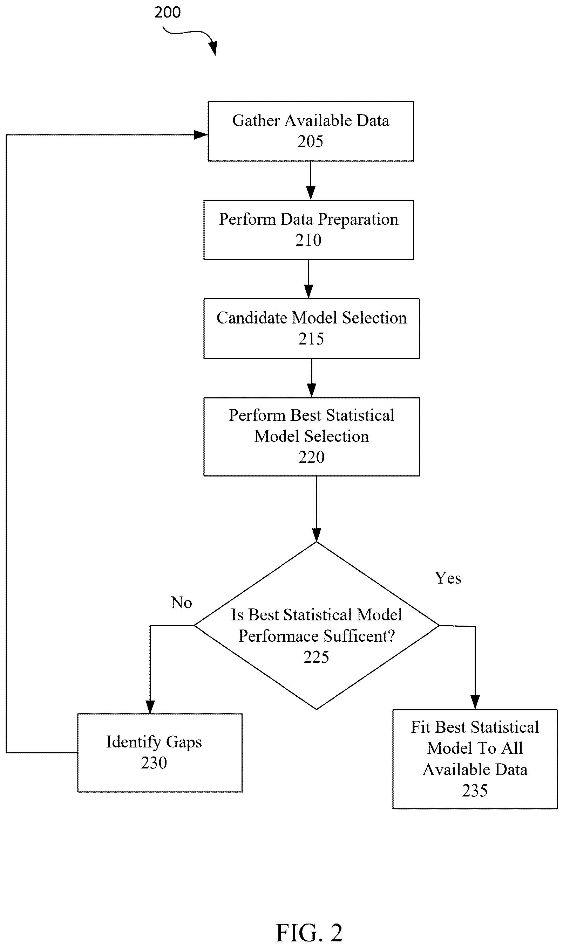

Figure 2 is another example of a method (200) of selecting and fitting a statistical model. Available data related to a metallic part is gathered (block 205). In some examples, the data is from the metallic part supplier. The supplier data contains the static mechanical property values and fracture toughness results for previously tested metallic parts. The merged datasets, that is, the static mechanical property dataset and the fracture toughness dataset, are then split into a training dataset and a test dataset.

Once the data is gathered, the data goes through a data preparation process (block 210). The data preparation includes merging the static mechanical property dataset and fracture toughness dataset and assigning a pass or fail indicator for each metallic part based on an engineering requirement. The full dataset is then split into a training dataset and a test dataset.

The training dataset is used to develop candidate models. One or more of the candidate models is selected from the statistical models (block 215). In some examples, a 10-fold cross validation and stepwise model selection is performed using AIC/BIC Criterion. The best statistical model is selected from the candidate models (block 220) and evaluated using the test dataset to determine if the performance of the selected model is sufficient (block 225). If there are identified gaps within the data (230) the process repeats. In some examples, the selected best statistical model is insufficient when it is not sufficiently able to classify parts with passing or failing fracture toughness or if there is insufficient data representation. In particular, the model may produce a higher than acceptable false positive rate (predicting a part to having a failing fracture toughness when it truly passed) or a false negative rate (predicting a part to have a passing fracture toughness when it truly failed) for the application. In other examples, when the AUC is not significantly larger than 0.5, then the model is not sufficient. 0.5 represents correctly predicting the outcome 50% of the time. If the best statistical model is sufficient, the full dataset is used to fit the selected best statistical model (block 235).

Once a fitted best statistical model is determined such as using one of the methods disclosed in Figures 1 or 2, the fitted best statistical model is used to predict the probability of fracture toughness failure in parts and determine which parts warrant additional testing based on a predicted probability of failure.

The number of parts that are actually tested in a lot can vary. In some examples, suppliers require that a minimum number of parts per heat treat lot be tested to cover the entire heat treat lot. The parts selected for testing may be random or predetermined based on criteria. For example, the number of fracture toughness samples may be a percentage of overall parts based on a heat treatment lot size. In some examples, a random sampling of parts is tested.

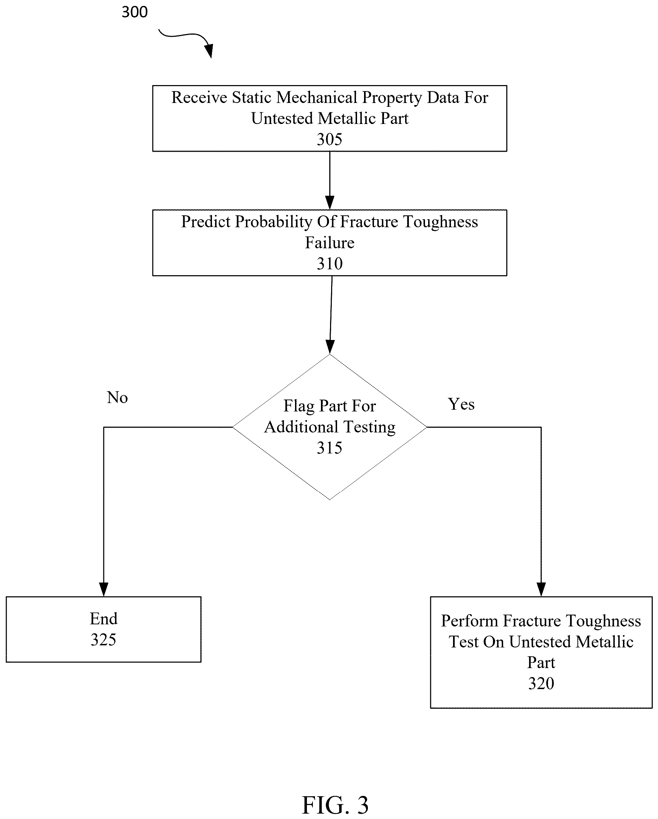

Figure 3 is a flowchart illustrating a method (300) for predicting the probability of fracture toughness failure. Once the static mechanical properties of untested parts are received (block 305), the fitted best statistical model is used to predict the probability of fracture toughness failure of the untested parts (block 310). The static mechanical property dataset of the untested parts is inputted into the fitted best statistical model. The fitted best statistical model predicts the probability of fracture toughness failure by outputting a probability and a confidence interval about that probability. In some examples, the normal approximation or Wald method is used to compute the confidence interval based on the desired confidence level the log-odds scale. This interval is then exponentiated to give the confidence interval on the probability scale.

Once the probability is predicted and the confidence interval about the probability is computed, the method determines whether additional testing is warranted (block 315). In some examples, this includes comparing the probability and/or upper confidence bound about that probability to a predetermined threshold. In some examples, the probability threshold for the classification of failure depends on the side of acceptable risk for the application. When the probability and/or upper confidence bound on that probability is over the predetermined threshold the untested part is predicted to fail a fracture toughness test. The flag may be triggered based on a probability threshold indicating that the untested part is likely to fail, and additional testing may be performed (320).

When the probability and/or upper confidence bound on that probability is under the predetermined threshold the untested part is predicted to pass a fracture toughness test, and further testing may not be required (block 325). In some examples, there could be other reasons to perform the testing (e.g., testing may be part of a sampling requirement imposed by a manufacture or purchaser).

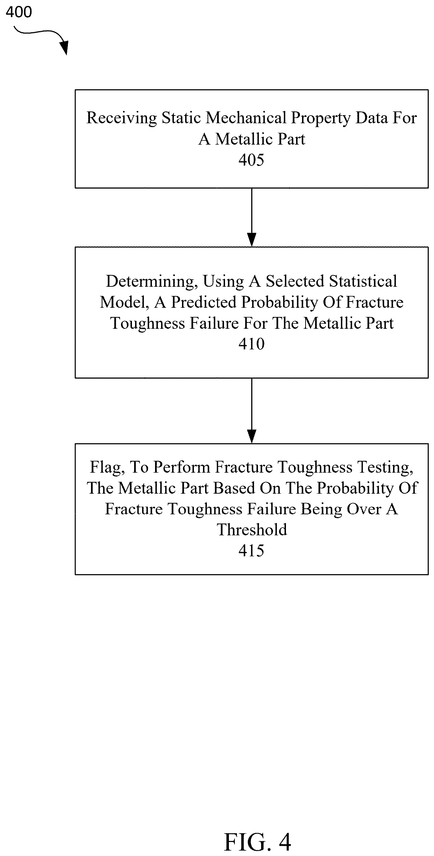

Figure 4 is a flowchart illustrating another method (400) for predicting the likelihood of fracture toughness failure of a metallic part. The method includes receiving static mechanical property data for a metallic part (block 405). In some examples, the metallic part is selected from a heat treat lot that includes multiple metallic parts. The static mechanical properties of the metallic part are tested to evaluate how the metallic part reacts to external forces, such as tensile force or compressive force, without undergoing permanent deformation or failure.

The method further includes determining a predicted probability of fracture toughness failure for the metallic part using a selected statistical model (block 410). In some examples, the selected statistical model is a fitted best statistical model described above. In some examples, the selected statistical model is a generalized linear model or logistic regression model that produces a predicted probability of fracture toughness failure. The selected statistical model is trained using a training dataset and a test dataset from previous test materials. The training dataset and test dataset include previous test metallic materials and fracture toughness data. In some examples, the full dataset is divided into a 75% training dataset and a 25% testing dataset split. The stored static mechanical property data includes pass and fail fracture toughness data of metallic parts that have previously undergone fracture toughness testing. In some examples, the selected statistical model is evaluated using user input. The user may be a subject matter expert.

The metallic part is flagged based on the predicted probability of fracture toughness failure over a threshold (block 415). The flagging indicates that the metallic part is predicted to have a fracture toughness failure and may need further fracture toughness testing. In some examples, the threshold is set based on user input obtained prior to predicting a probability of fracture toughness failure.

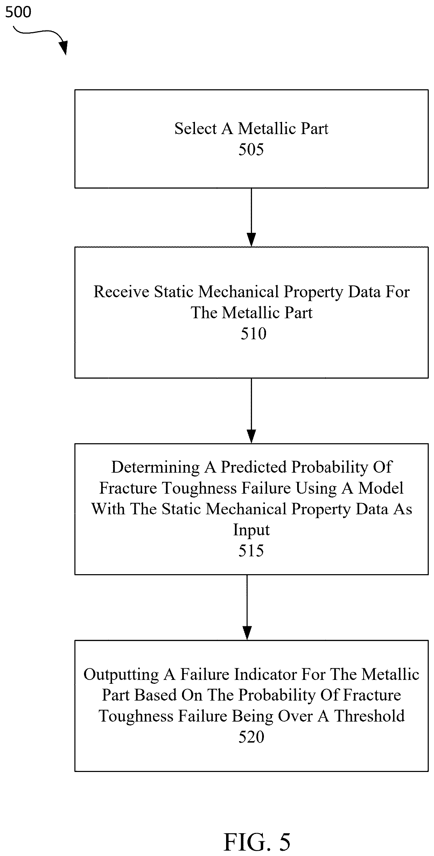

Figure 5 is a flowchart illustrating a method 500 for predicting fracture toughness failure. A method of predicting the fracture toughness failure of a metallic part includes selecting a metallic part (block 505). The metallic part may be selected from a heat treat lot including multiple metallic parts.

The method further includes receiving static mechanical property data for the metallic part (block 510). The static mechanical properties of the metallic part are tested to evaluate how the metallic part reacts to external forces, such as tensile force or compressive force, without undergoing permanent deformation or failure.

The method further includes determining a predicted probability of fracture toughness failure using a model with the static mechanical property data as input (515). In some examples, the model is built using stored static mechanical property data and fracture toughness data from previously tested metallic parts. In some examples, the model is a statistical model.

The method further includes outputting a failure indicator for the metallic part based on the probability of fracture toughness failure being over the threshold (520). In some examples, the model produces a predicted probability of fracture toughness failure. For example, the failure indicator may indicate a predicted pass or fail signal. In some examples, logistic regression is used to model the fracture toughness pass and failure data.

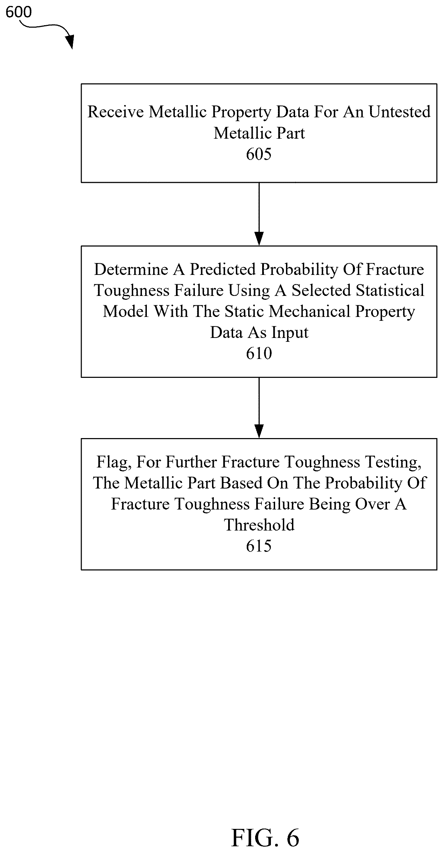

Figure 6 is a flowchart illustrating a method 600 for predicting fracture toughness failure. The fracture toughness failure is predicted by a non-transitory computer-readable medium storing a computer program product for controlling a computing device. The computer program product comprises software instructions that, when run on the computing device, cause the computing device to receive static mechanical property data for an untested metallic part (block 605). The computing device is further caused to determine a predicted probability of fracture toughness using a selected statistical model with the static mechanical property data as input (block 610). The computing device is also caused to flag, for further fracture toughness testing, the metallic part based on the probability of fracture toughness failure being over a threshold (block 615).

In some examples, the metallic part is selected from a heat treat lot that includes multiple parts. In some examples, the static mechanical property data for a metallic part comprises pass and fail fracture toughness data of previously undergone fracture toughness testing. In some examples, the static mechanical property data comprises data indicating compression, ultimate tensile strength, yield strength, percent elongation, percent reduction of area, and hardness after aging of the untested metallic part. In some examples, the static mechanical property data comprises data indicating compression, ultimate tensile strength, yield strength, percent elongation, percent reduction of area, and hardness after aging of a tested metallic part.

The methods disclosed herein provide multiple benefits. The disclosed approach allows for quickly identifying suspect metallic parts in the event of a nonconformance with mechanical standards or could be used alongside static mechanical property testing to identify which metallic parts of a given heat treat lot should be selected for fracture toughness testing. In this approach, only a sample of metallic parts is tested. As a result, this resolves the time and cost burden associated with performing fracture toughness testing on all metallic parts and allows for rapid identification of suspect parts with respect to fracture toughness. This approach can also be adapted to allow for ongoing refinement, including incorporating additional static mechanical property test dataset as part of engineering requirements into the statistical model and expanding the model to include additional part numbers or effects that may impact fracture toughness failure (heat treat lot, coupon location, furnace, etc.). Suppliers can also use this approach to identify heat treat lots based on risk from a heat treat lot for fracture toughness sampling. The prediction supports nonconformance issues where fracture toughness would substantiate the recommendation to accept parts and reduce the risks to a company of accepting poor quality parts.

Methods 100, 200, 300, and 400, as illustrated in Figures 1-4, may be performed by a computing device 750.

A non-transitory computer-readable medium storing a computer program product for controlling a computing device 750, the computer program product comprising software instructions that, when run on the computing device 750, may cause the computing device 750 to perform any of the methods 100, 200, 300, and 400.

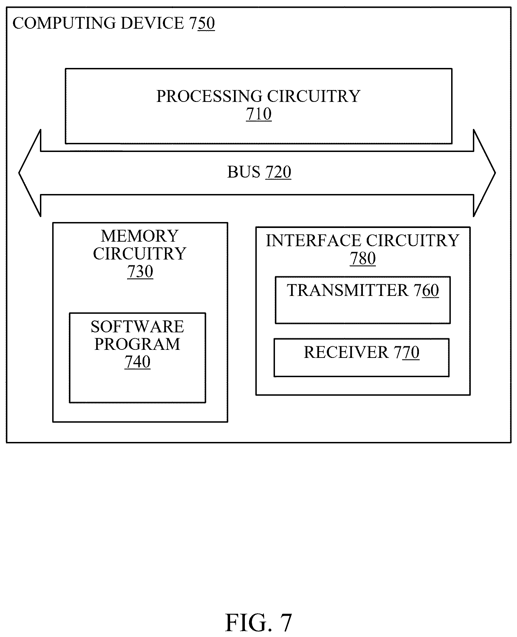

FIG. 7 is a schematic block diagram illustrating an example of a computing device 750. The computing device 750 includes processing circuitry 710, memory circuitry 730, and interface circuitry 780. The processing circuitry 710 is communicatively coupled to the memory circuitry 730 and the interface circuitry 780, e.g., via one or more buses 720. The processing circuitry 710 includes one or more microprocessors, microcontrollers, hardware circuits, discrete logic circuits, hardware registers, digital signal processors (DSPs), field-programmable gate arrays (FPGAs), application-specific integrated circuits (ASICs), or a combination thereof. In one example, the processing circuitry 710 is programmable hardware capable of executing one or more software programs 740 stored, e.g., as a machine-readable computer program in the memory circuitry 730.

The memory circuitry 730 includes non-transitory machine-readable media, whether volatile or non-volatile, including but not limited to solid state media (e.g., SRAM, DRAM, DDRAM, ROM, PROM, EPROM, flash memory, solid-state drive, etc.), removable storage devices (e.g., Secure Digital (SD) card, miniSD card, microSD card, memory stick, thumb-drive, USB flash drive, ROM cartridge, Universal Media Disc), fixed drive (e.g., magnetic hard disk drive), or the like, wholly or in various combination. The memory circuitry 730 may store one or more software programs 740.

In some examples, the interface circuitry 780 is a controller hub configured to control the input and output (I/O) data paths of the computing device 750. Such I/O data paths may include data paths for exchanging signals over a communications network and data paths for exchanging signals with an electronic device or a user. For example, interface circuitry 780 includes a transceiver configured to send and receive communication signals over one or more of a wireless network, Ethernet network, or optical network. In some examples, the interface circuitry 780 includes (or is communicatively connected to) one or more graphics adapter, display port, video bus, touchscreen, graphical processing unit (GPU), display port, Liquid Crystal Display (LCD), and Light Emitting Diode (LED) display, for presenting visual information to a user. In some examples, the interface circuitry 780 includes one or more of a pointing device (e.g., a mouse, stylus, touchpad, trackball, pointing stick, joystick), touchscreen, microphone for speech input, optical sensor for optical recognition of gestures, and keyboard for text entry.

The interface circuitry 780 may be implemented as a unitary physical component or as a plurality of physical components that are contiguously or separately arranged, any of which may be communicatively coupled to any other or may communicate with any other via the processing circuitry 710. In one example, the interface circuitry 780 includes output circuitry (e.g., transmitter 760 configured to send communication signals over the communications network) and input circuitry (e.g., receiver 770 configured to receive communication signals over the communications network). Similarly, the output circuitry may include a display, whereas the input circuitry may include a keyboard, touch screen, or card reader. Other examples, permutations, and arrangements of the above and their equivalents will be readily apparent to those of ordinary skill.

The present disclosure may, of course, be carried out in ways other than those specifically set forth herein without departing from the essential characteristics of the disclosure. The present embodiments are to be considered in all respects as illustrative and not restrictive, and all changes coming within the meaning and equivalency range of the appended claims are intended to be embraced therein.

Further, the present disclosure comprises aspects according to the following clauses.
Clause 1. A method of predicting a probability of fracture toughness failure of a metallic part, the method comprising:
   receiving static mechanical property data for the metallic part;
   determining, using a selected statistical model, a predicted probability of fracture toughness failure for the metallic part; and
   flagging, to perform fracture toughness testing, the metallic part based on the predicted probability of fracture toughness failure being over a threshold.
Clause 2. The method of clause 1, wherein the selected statistical model is a generalized linear model or logistic regression model.
Clause 3. The method of clause 1, further comprising developing the selected statistical model using a training dataset and a test dataset with the training dataset and the test dataset comprising a stored static mechanical property data and fracture toughness data.
Clause 4. The method of clause 3, wherein, a full dataset is split into a 75% training dataset and a 25% test dataset.
Clause 5. The method of clause 3, wherein the static mechanical property data comprises pass and fail fracture toughness data of metallic parts that have previously undergone fracture toughness testing based on engineering requirements.
Clause 6. The method of clause 1, further comprising selecting the metallic part from a heat treat lot comprising multiple metallic parts.
Clause 7. The method of clause 1, further comprising evaluating the selected statistical model using user input.
Clause 8. The method of clause 1, further comprising setting the threshold based on user input obtained prior to predicting the probability of fracture toughness failure.
Clause 9. The method of clause 1, wherein the flagging the metallic part is responsive to determining that the metallic part is likely to fail an engineering requirement for fracture toughness.
Clause 10. A method of predicting a probability of fracture toughness failure of a metallic part, the method comprising:
   selecting the metallic part;
   receiving static mechanical property data for the metallic part;
   determining a predicted probability of fracture toughness failure using a model with the static mechanical property data as input; and
   outputting a failure indicator for the metallic part based on the probability of fracture toughness failure being over a threshold.
Clause 11. The method of clause 10, wherein the static mechanical property data indicates how the metallic part reacts to external forces, such as pulling, compression or shearing.
Clause 12. The method of clause 10, wherein the model is developed using stored paired static mechanical property data and fracture toughness data from previously tested metallic parts.
Clause 13. The method of clause 10 wherein the metallic part is selected from a heat treat lot comprising multiple metallic parts.
Clause 14. The method of clause 10, wherein the model is a statistical model.
Clause 15. The method of clause 10 wherein, the model produces the predicted probability of fracture toughness failure.
Clause 16. The method of clause 10, wherein logistic regression is used to model a binary outcome of a fracture toughness test with respect to an engineering requirement.
Clause 17. A non-transitory computer-readable medium storing a computer program product for controlling a computing device, the computer program product comprises software instructions that, when run on the computing device, causes the computing device to:
   receive static mechanical property data for an untested metallic part;
   determine a predicted probability of fracture toughness failure using a selected statistical model with the static mechanical property data as input; and
   flag, for further fracture toughness testing, the untested metallic part based on the predicted probability of fracture toughness failure being over a threshold.
Clause 18. The non-transitory computer-readable medium of clause 17, wherein the untested metallic part is selected from a heat treat lot including multiple parts.
Clause 19. The non-transitory computer-readable medium of clause 17, wherein the static mechanical property data for the untested metallic part comprises pass and fail fracture toughness data of previously undergone fracture toughness testing.
Clause 20. The non-transitory computer-readable medium of clause 17, wherein the static mechanical property data comprises data indicating compression, ultimate tensile strength, yield strength, percent elongation, percent reduction of area, and hardness after aging of a tested or the untested metallic part.

## Claims

1. A method of predicting a probability of fracture toughness failure of a metallic part, the method comprising:
selecting the metallic part;
receiving (405) static mechanical property data for the metallic part;
determining (410), using either a selected statistical model, or a model with the static mechanical property data as input, a predicted probability of fracture toughness failure for the metallic part; and
either flagging (415), to perform fracture toughness testing, the metallic part, or outputting a failure indicator for the metallic part based on the predicted probability of fracture toughness failure being over a threshold.

2. The method of claim 1, wherein the selected statistical model is a generalized linear model or logistic regression model.

3. The method of any one of the preceding claims, further comprising developing the selected statistical model using a training dataset and a test dataset with the training dataset and the test dataset comprising a stored static mechanical property data and fracture toughness data.

4. The method of claim 3, wherein, a full dataset is split into a 75% training dataset and a 25% test dataset.

5. The method of any one of the preceding claims, wherein the static mechanical property data comprises pass and fail fracture toughness data of metallic parts that have previously undergone fracture toughness testing based on engineering requirements.

6. The method of any one of the preceding claims, further comprising selecting the metallic part from a heat treat lot comprising multiple metallic parts.

7. The method of any one of the preceding claims, further comprising evaluating the selected statistical model using user input.

8. The method of any one of the preceding claims, further comprising setting the threshold based on user input obtained prior to predicting the probability of fracture toughness failure.

9. The method of any one of the preceding claims, wherein the flagging the metallic part is responsive to determining that the metallic part is likely to fail an engineering requirement for fracture toughness.

10. The method of any one of the preceding claims, wherein the static mechanical property data indicates how the metallic part reacts to external forces, such as pulling, compression or shearing.

11. The method of any one of the preceding claims, wherein the model is developed using stored paired static mechanical property data and fracture toughness data from previously tested metallic parts.

12. The method of claim 1, wherein the model is a statistical model.

13. The method of any one of the preceding claims, wherein, the model produces the predicted probability of fracture toughness failure.

14. The method of any one of the preceding claims, wherein logistic regression is used to model a binary outcome of a fracture toughness test with respect to an engineering requirement.

15. A non-transitory computer-readable medium storing a computer program product for controlling a computing device, the computer program product comprises software instructions that, when run on the computing device, causes the computing device to carry out a method according to any one of the preceding claims.
